(19) Europäisches Patentamt
European Patent Office
Office européen des brevets

(11) Veröffentlichungsnummer : **0 143 384**
**B1**

(12) # EUROPÄISCHE PATENTSCHRIFT

(45) Veröffentlichungstag der Patentschrift : **05.04.89**

(51) Int. Cl.⁴ : **C 07 D249/08**

(21) Anmeldenummer : **84113617.9**

(22) Anmeldetag : **12.11.84**

(54) **Verfahren zur Herstellung von Beta-Hydroxyethyl-(1,2,4-triazol)-Derivaten.**

(30) Priorität : **25.11.83 DE 3342693**

(43) Veröffentlichungstag der Anmeldung : **05.06.85 Patentblatt 85/23**

(45) Bekanntmachung des Hinweises auf die Patenterteilung : **05.04.89 Patentblatt 89/14**

(84) Benannte Vertragsstaaten :
**BE CH DE FR GB IT LI NL**

(56) Entgegenhaltungen :
**J. HETEROCYCLIC CHEM., Band 17, Nr. 6, 1980, Seite 1319, HeteroCorporation; S.D. ZIMAN: "A new mesoionic species in the synthesis of some 5-thioxo-1,2,4-triazolin-3-ones"**
**JOURNAL OF THE CHEMICAL SOCIETY PERKIN TRANSACTIONS I ORGANIC AND BIO-ORGANIC CHEMISTRY, Nr. 5, 1984, Seiten 993-998; S.P. LANGDON: "Triazines and related products. Part 26.1 Synthesis and chemistry of bicyclic analogues of the antitumour drug 2,4,6-tris(dimethylamino)-1,3,5-triazine (hexamethylmelamine)"**

(73) Patentinhaber : **BAYER AG**
**Konzernverwaltung RP Patentabteilung**
**D-5090 Leverkusen 1 Bayerwerk (DE)**

(72) Erfinder : **Lantzsch, Reinhard, Dr.**
**Heymannstrasse 32**
**D-5090 Leverkusen 1 (DE)**
Erfinder : **Reubke, Karl-Julius, Dr.**
**Rybniker Strasse 10**
**D-5000 Köln 80 (DE)**

**EP 0 143 384 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von bekannten β-Hydroxyethyl-(1,2,4-triazol)-Derivaten, welche pflanzenwuchsregulierende und fungizide Eigenschaften besitzen.

Es ist bereits bekannt geworden, daß man β-Hydroxyethyl-(1,2,4-triazol)-Derivate herstellen kann, indem man Oxirane mit 1,2,4-Triazol in Gegenwart einer Base sowie in Gegenwart von inerten Lösungsmitteln umsetzt (vgl. EP-A 40 345, EP-A 44 605, EP-A 46 337, EP-A 47 594, EP-A 52 424 und DE-A 26 54 890). Nachteilig an diesem Verfahren ist jedoch, daß sich außer den 1,2,4-Triazol-Derivaten je nach dem verwendeten Solvens mehr oder weniger große Mengen an 1,3,4-Triazol-Derivaten bilden. Arbeitet man beispielsweise in Gegenwart von Alkoholen, so entstehen bis zu 30 % an den unerwünschten Stoffen. Die Abtrennung dieser störenden Nebenprodukte ist aufwendig, und die Ausbeuten an den gewünschten 1,2,4-Triazol-Derivaten liegen daher nur relativ niedrig.

Es wurde nun gefunden, daß man die bekannten β-Hydroxyethyl-(1,2,4-triazol)-Derivate der Formel

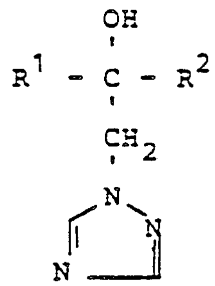

(I)

in welcher

$R^1$ für geradkettiges oder verzweigtes Alkyl mit 1 bis 8 Kohlenstoffatomen, geradkettiges oder verzweigtes Halogenalkyl mit 1 bis 4 Kohlenstoffatomen und 1 bis 5 Halogenatomen, Alkenyl mit 2 bis 8 Kohlenstoffatomen, Alkinyl mit 2 bis 8 Kohlenstoffatomen, gegebenenfalls durch Alkyl mit 1 oder 2 Kohlenstoffatomen substituiertes Cycloalkyl mit 3 bis 7 Kohlenstoffatomen, gegebenenfalls durch Alkyl mit 1 oder 2 Kohlenstoffatomen substituiertes Cycloalkenyl mit 5 bis 8 Kohlenstoffatomen, oder für Phenyl steht, das einfach bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes.

Phenylalkyl mit 1 oder 2 Kohlenstoffatomen im Alkylteil und gegebenenfalls durch Halogen und/oder Alkyl mit 1 bis 4 Kohlenstoffatomen substituiertes Phenylalkoxy mit 1 oder 2 Kohlenstoffatomen im Alkoxyteil,

oder

$R^1$ für Phenylalkyl mit 1 bis 4 Kohlenstoffatomen im Alkylteil steht, wobei der Phenylalkyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil,

oder

$R^1$ für Phenylalkenyl mit 2 bis 4 Kohlenstoffatomen im Alkenylteil steht, wobei der Phenylalkenyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy,

gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil, oder

$R^1$ für einen gegebenenfalls durch Halogen und/oder Alkyl mit 1 bis 4 Kohlenstoffatomen substituierten heterocyclischen Rest mit 5 bis 7 Ring-Gliedern und 1 bis 3 Heteroatomen steht
und

$R^2$ für Wasserstoff, geradkettiges oder verzweigtes Alkyl mit 1 bis 8 Kohlenstoffatomen, geradkettiges oder verzweigtes Halogenalkyl mit 1 bis 4 Kohlenstoffatomen und 1 bis 5 Halogenatomen, Alkenyl mit 2 bis 8 Kohlenstoffatomen, Alkinyl mit 2 bis 8 Kohlenstoffatomen, gegebenenfalls durch Alkyl mit 1 oder 2 Kohlenstoffatomen substituiertes Cycloalkyl mit 3 bis 7 Kohlenstoffatomen, gegebenenfalls durch Alkyl mit 1 oder 2 Kohlenstoffatomen substituiertes Cycloalkenyl mit 5 bis 8 Kohlenstoffatomen steht oder für Phenyl steht, das einfach bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und gegebenenfalls durch Halogen und/oder Alkyl mit 1 bis 4 Kohlenstoffatomen substituiertes Phenylalkoxy mit 1 oder 2 Kohlenstoffatomen im Alkoxyteil,
oder

$R^2$ für Phenylalkyl mit 1 bis 4 Kohlenstoffatomen im Alkylteil steht, wobei der Phenylalkyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil,
oder

$R^2$ für Phenylalkenyl mit 2 bis 4 Kohlenstoffatomen im Alkenylteil steht, wobei der Phenylalkenyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil,
oder

$R^2$ für einen gegebenenfalls durch Halogen und/oder Alkyl mit 1 bis 4 Kohlenstoffatomen substituierten heterocyclischen Rest mit 5 bis 7 Ring-Gliedern und 1 bis 3 Heteroatomen steht, erhält, wenn man β-Hydroxyethyl-(1,3,4-triazol)-Derivate der Formel

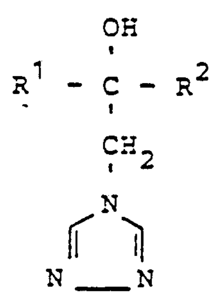

(II)

in welcher

$R^1$ und $R^2$ die oben angegebenen Bedeutungen haben,

in Gegenwart einer Base und in Gegenwart eines aprotischen, dipolaren Verdünnungsmittels sowie

gegebenenfalls in Gegenwart eines Co-Katalysators und gegebenenfalls in Gegenwart von Wasser bei Temperaturen zwischen 50 °C und 200 °C verrührt.

Es ist als äußerst überraschend zu bezeichnen, daß sich die Verbindungen der Formel (II) nach dem erfindungsgemäßen Verfahren in β-Hydroxyethyl-(1,2,4-triazol)-Derivate der Formel (I) überführen lassen, denn eine derartige Umlagerung war aus dem Stand der Technik noch nicht bekannt.

Das erfindungsgemäße Verfahren zeichnet sich durch eine Reihe von Vorteilen aus. So ermöglicht es die Herstellung von β-Hydroxyethyl-(1,2,4-triazol)-Derivaten der Formel (I) in extrem hohen Ausbeuten, wobei als Ausgangsprodukte solche Substanzen Verwendung finden, die bisher nur als störende Nebenprodukte anfielen und abgetrennt werden mußten. Ferner ist die Umsetzung einfach durchführbar, und die Isolierung der Verbindungen der Formel (I) bereitet keinerlei Schwierigkeiten. Besonders günstig ist hierbei, daß eine Umkristallisation der anfallenden Produkte nicht erforderlich ist.

Verwendet man 1-(4-Chlor-phenyl)-4,4-dimethyl-3-(1,3,4-triazol-1-yl-methyl)-pentan-3-ol als Ausgangsstoff, Natriumhydroxid als Base und N-Methylpyrrolidon als Verdünnungsmittel, so kann der Verlauf des erfindungsgemäßen Verfahrens durch das folgende Formelschema veranschaulicht werden :

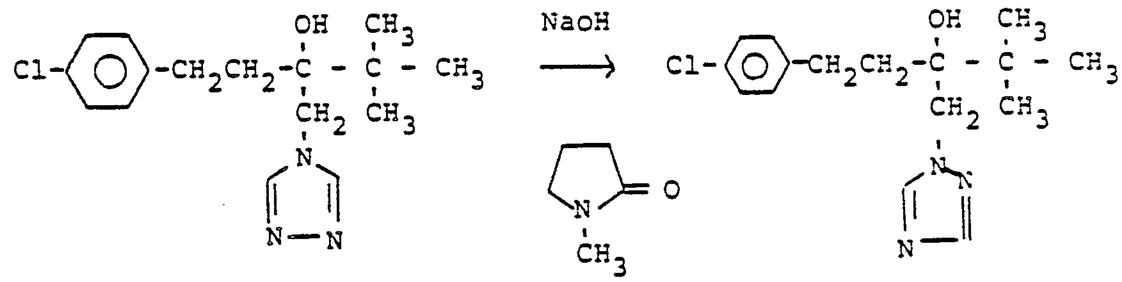


Die bei dem erfindungsgemäßen Verfahren als Ausgangsstoffe zu verwendenden β-Hydroxyethyl-(1,3,4-triazol)-Derivate sind durch die Formel (II) allgemein definiert.

Bevorzugte Ausgangsstoffe sind diejenigen Verbindungen der Formel (II), in denen

$R^1$ für Methyl, Ethyl, Propyl, Isopropyl, n-Butyl, iso-Butyl, sek.-Butyl, tert.-Butyl, durch 1 bis 3 Fluor- und/oder Chloratome substituiertes Methyl, Ethyl, Propyl, Isopropyl, n-Butyl, iso-Butyl, sek.-Butyl oder tert.-Butyl steht, ferner für Alkenyl mit 2 bis 6 Kohlenstoffatomen, Alkinyl mit 2 bis 6 Kohlenstoffatomen, gegebenenfalls durch Methyl substituiertes Cyclopropyl, Cyclopentyl oder Cyclohexyl, gegebenenfalls durch Methyl substituiertes Cyclopentenyl, Cyclohexenyl, Cycloheptenyl oder Cyclooctenyl steht, weiterhin für Phenyl steht, welches 1 bis dreifach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder gegebenenfalls durch Fluor, Chlor und/oder Methyl substituiertes Benzyloxy, und außerdem für Phenethyl steht, das im Phenylteil 1 bis 3-fach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor, und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder gegebenenfalls durch Fluor, Chlor und/oder Methyl substituiertes Benzyloxy, und weiterhin auch für 2-Phenyl-ethenyl steht, das im Phenylteil 1 bis 3-fach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder gegebenenfalls durch Fluor, Chlor und/oder Methyl substituiertes Benzyloxy, und außerdem für gegebenenfalls durch Chlor und/oder Methyl substituiertes Furanyl, Thiophenyl, Pyridinyl, Pyrimidinyl und Pyrrolyl steht, und

$R^2$ für Wasserstoff, Methyl, Ethyl, Propyl, Isopropyl, n-Butyl, iso-Butyl, sek.-Butyl, tert.-Butyl, durch 1 bis 3 Fluor- und/oder Chloratome substituiertes Methyl, Ethyl, Propyl, Isopropyl, n-Butyl, iso-Butyl, sek.-Butyl oder tert.-Butyl steht, ferner für Alkenyl mit 2 bis 6 Kohlenstoffatomen, Alkinyl mit 2 bis 6 Kohlenstoffatomen, gegebenenfalls durch Methyl substituiertes Cyclopropyl, Cyclopentyl oder Cyclohexyl, gegebenenfalls durch Methyl substituiertes Cyclopentenyl, Cyclohexenyl, Cycloheptenyl oder Cyclooctenyl steht, weiterhin für Phenyl steht, welches 1 bis dreifach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder gegebenenfalls durch Fluor, Chlor und/oder Methyl substituiertes Benzyloxy, und außerdem für Phenethyl steht, das im Phenylteil 1 bis 3-fach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls

durch Fluor, Chlor und/oder Methyl substituiertes Benzyl und/oder gegebenenfalls durch Fluor Chlor und/oder Methyl substituiertes Benzyloxy, und weiterhin auch für 2-Phenyl-ethenyl steht, das im Phenylteil 1 bis 3-fach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder gegebenenfalls durch Fluor, Chlor und/oder Methyl substituiertes Benzyloxy, und außerdem für gegebenenfalls durch Chlor und/oder Methyl substituiertes Furanyl, Thiphenyl, Pyridinyl, Pyrimidinyl und Pyrrolyl steht.

Als Beispiele für β-Hydroxyethyl-(1,3,4-triazol)-Derivate der Formel (II) seien die in der folgenden Tabelle formelmäßig aufgeführten Stoffe genannt.

Tabelle 1

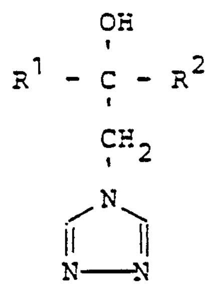

(II)

| $R^1$ | $R^2$ |
|---|---|
| 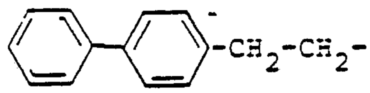 | $-C(CH_3)_3$ |
| 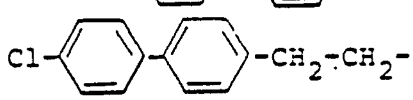 | " |
| 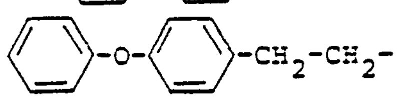 | " |
| 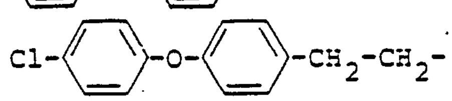 | " |
| 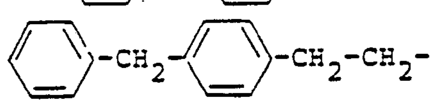 | " |
| 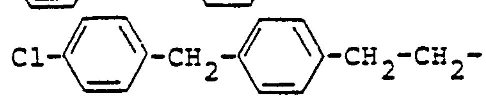 | " |
| 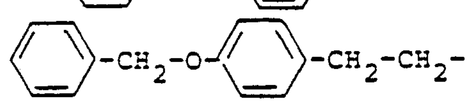 | " |
| 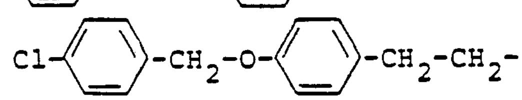 | " |
| 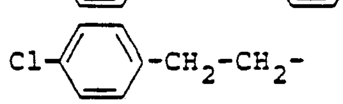 | " |
| 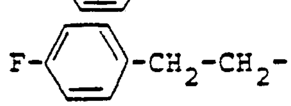 | " |
| 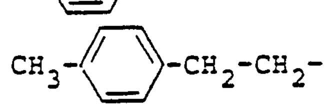 | " |
| 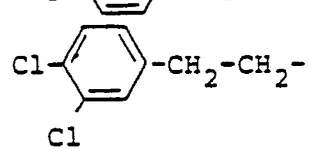 | " |

Tabelle 1 (Fortsetzung)

| $R^1$ | $R^2$ |
|---|---|
| 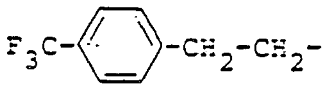 | $-C(CH_3)_3$ |
| 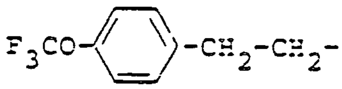 | " |
| 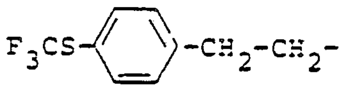 | " |
| 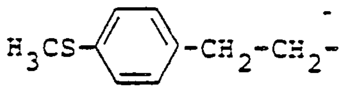 | " |
| 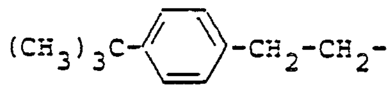 | " |
| 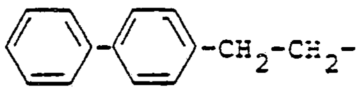 | 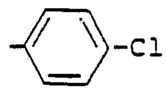 |
| 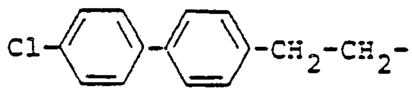 | " |
| 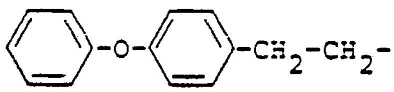 | " |
| 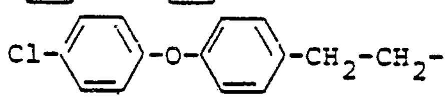 | " |
| 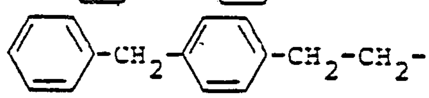 | " |
| 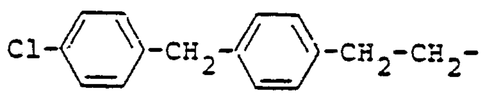 | " |
| 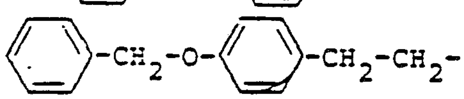 | " |
| 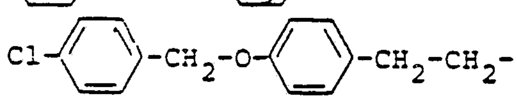 | " |
| 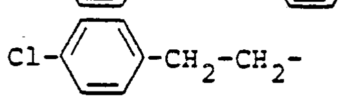 | " |
| 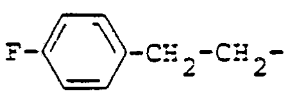 | 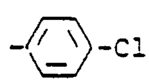 |
| 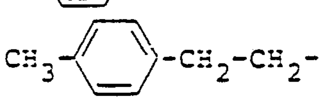 | " |
| 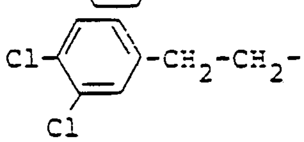 | " |
| 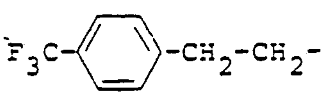 | " |
| 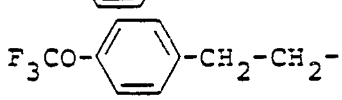 | " |

Tabelle 1 (Fortsetzung)

| $R^1$ | $R^2$ |
|---|---|
| 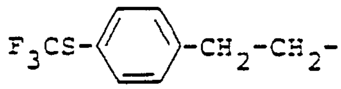 | " |
| 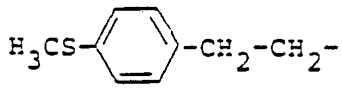 | " |
| 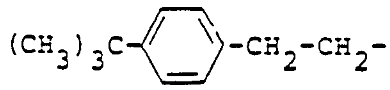 | " |
| 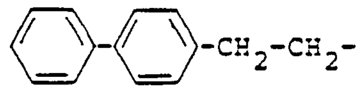 | $-CH(CH_3)_2$ |
| 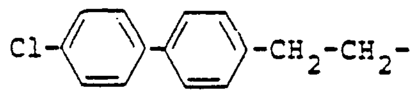 | " |
| 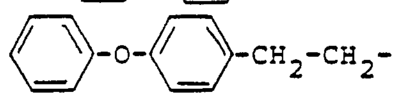 | " |
| 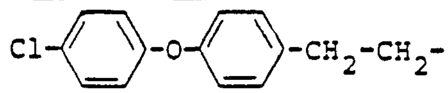 | " |
| 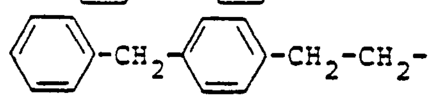 | " |
| 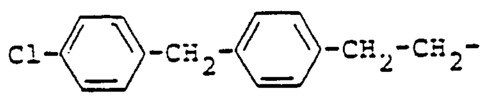 | $-CH(CH_3)_2$ |
| 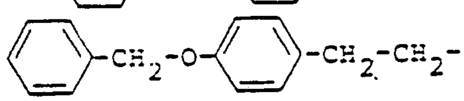 | " |
| 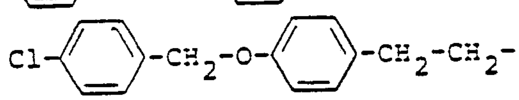 | " |
| 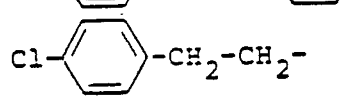 | " |
| 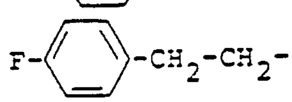 | " |
| 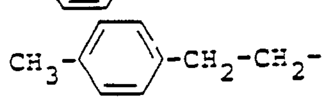 | " |
| 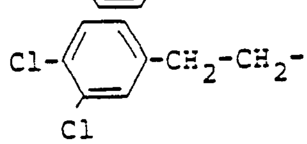 | " |
| 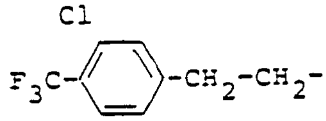 | " |
| 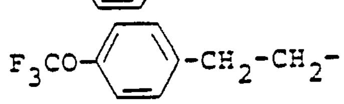 | " |
| 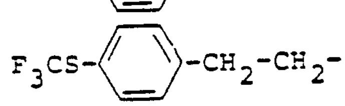 | " |
| 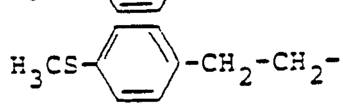 | " |

Tabelle 1 (Fortsetzung)

| $R^1$ | $R^2$ |
|---|---|
| 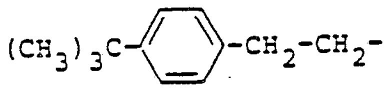 | " |
| 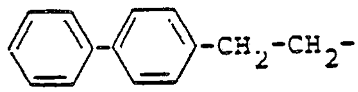 | 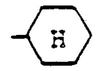 |
| 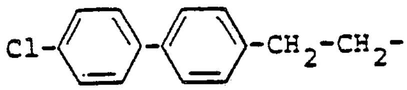 | " |
| 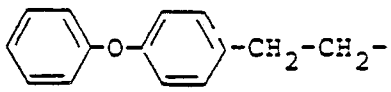 | 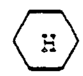 |
| 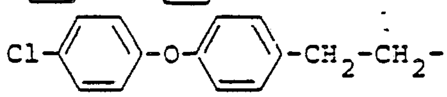 | " |
| 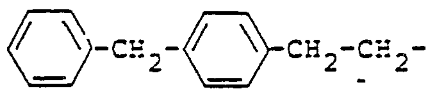 | " |
| 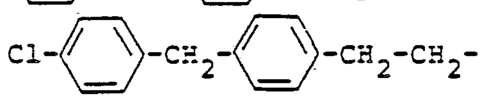 | " |
| 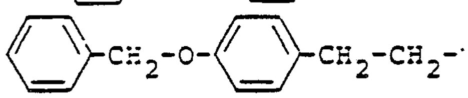 | " |
| 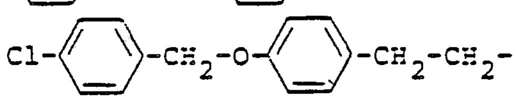 | " |
| 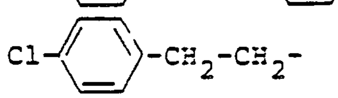 | " |
| 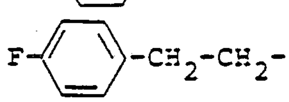 | " |
| 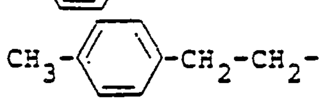 | " |
| 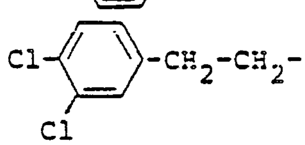 | " |
| 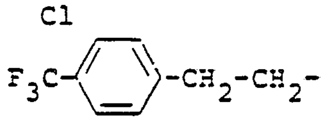 | " |
| 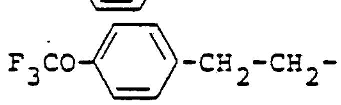 | " |
| 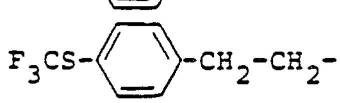 | " |
| 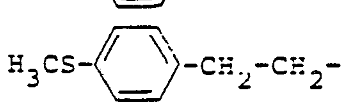 | 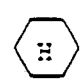 |
| 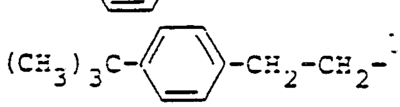 | " |
| 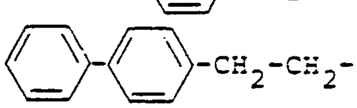 | 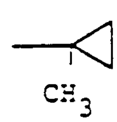 |

Tabelle 1 (Fortsetzung)

| $R^1$ | $R^2$ |
|---|---|
| 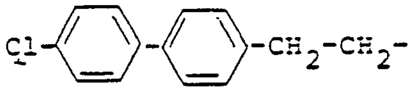 | " |
| 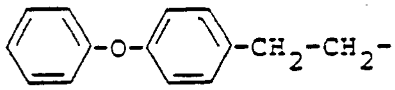 | " |
| 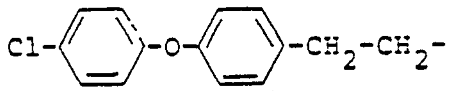 | " |
| 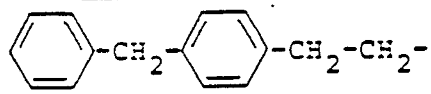 | " |
| 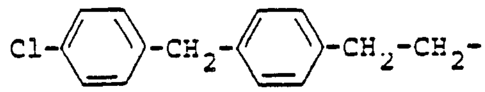 | " |
| 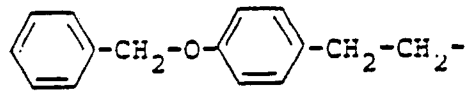 | " |
| 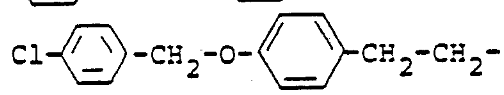 | " |
| 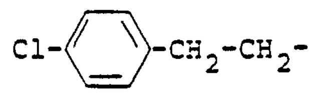 | " |
| 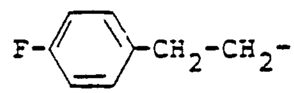 | " |
| 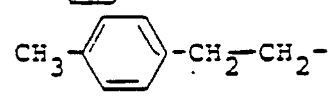 | " |
| 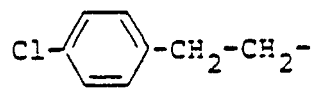 | 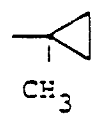 |
| 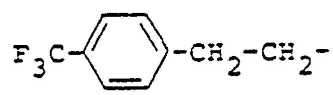 | " |
| 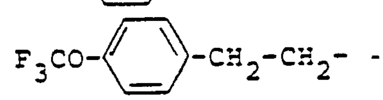 | " |
| 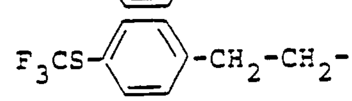 | " |
| 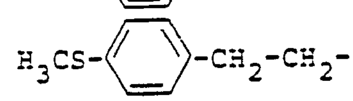 | " |
| 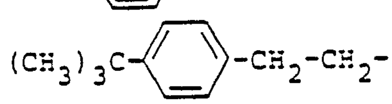 | " |
| 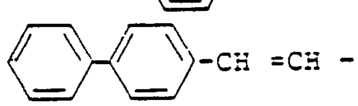 | $-C(CH_3)_3$ |
| 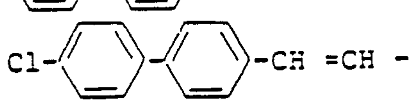 | " |
| 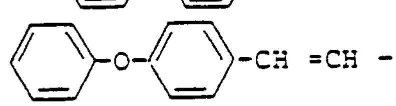 | " |

Tabelle 1 (Fortsetzung)

| $R^1$ | $R^2$ |
|---|---|
| 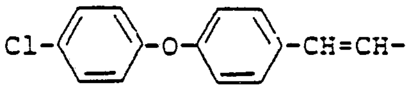 | " |
| 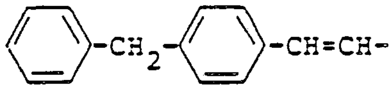 | " |
| 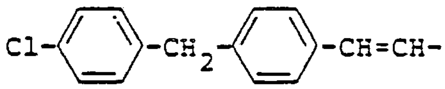 | " |
| 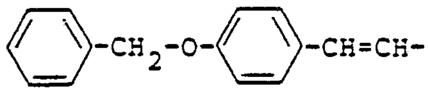 | " |
| 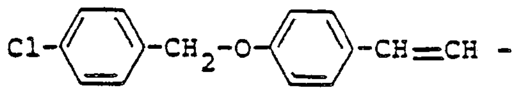 | " |
| 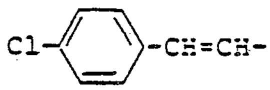 | $-C(CH_3)_3$ |
| 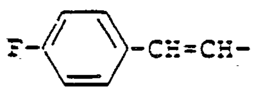 | " |
| 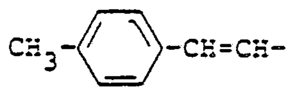 | " |
| 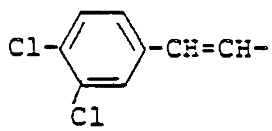 | " |
| 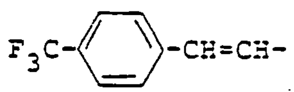 | " |
| 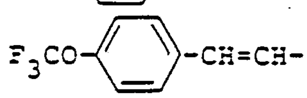 | " |
| 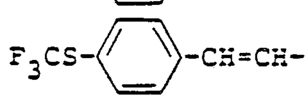 | " |
| 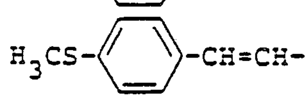 | " |
| 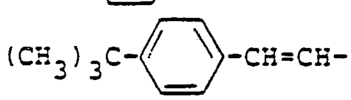 | " |
| 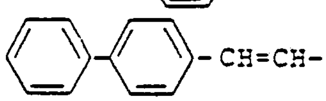 | 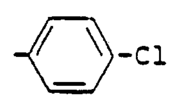 |
| 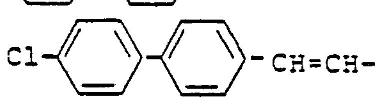 | " |
| 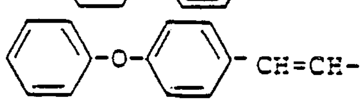 | " |
| 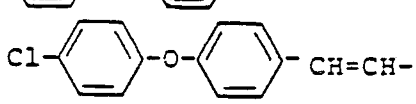 | " |

Tabelle 1 (Fortsetzung)

| $R^1$ | $R^2$ |
|---|---|
| 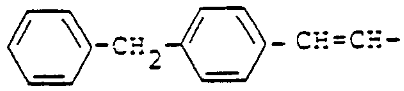 | 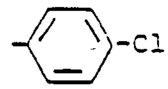 |
| 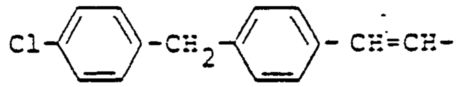 | " |
| 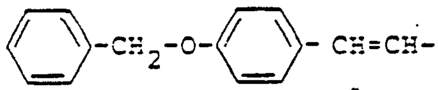 | " |
| 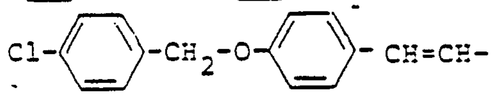 | " |
| 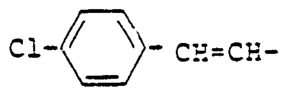 | " |
| 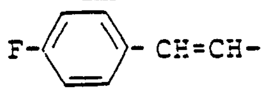 | " |
| 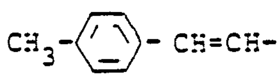 | " |
| 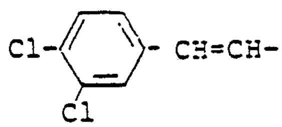 | " |
| 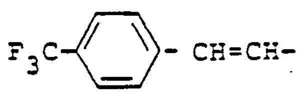 | " |
| 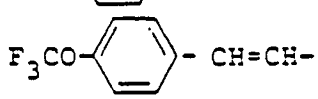 | " |
| 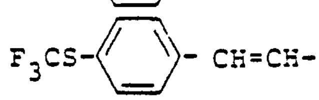 | " |
| 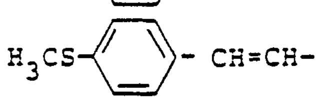 | " |
| 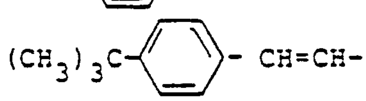 | " |
| 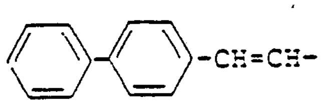 | $-CH(CH_3)_2$ |
| 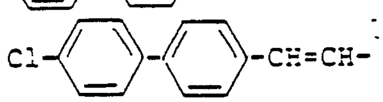 | " |
| 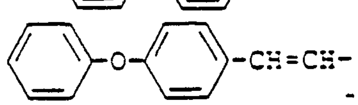 | " |
| 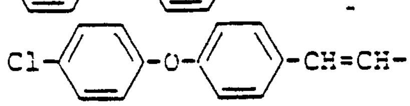 | " |
| 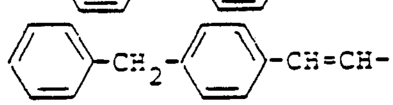 | " |
| 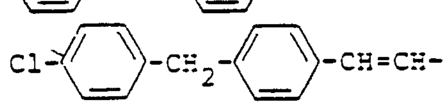 | " |

Tabelle 1 (Fortsetzung)

| $R^1$ | $R^2$ |
|---|---|
| 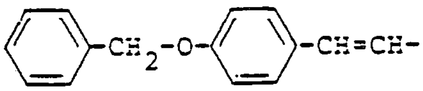 | " |
| 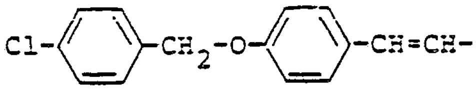 | " |
| 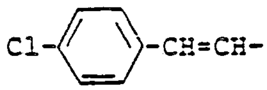 | " |
| 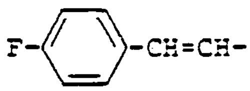 | " |
| 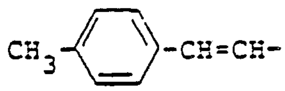 | " |
| 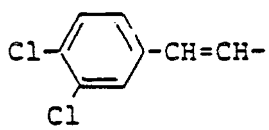 | " |
| 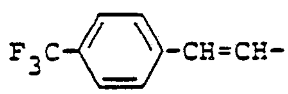 | " |
| 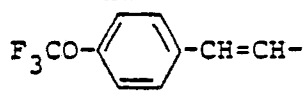 | " |
| 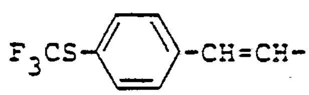 | $-CH(CH_3)_2$ |
| 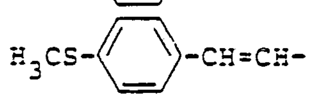 | " |
| 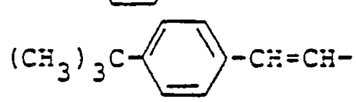 | " |
| 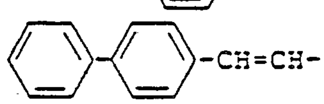 | 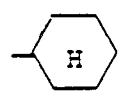 |
| 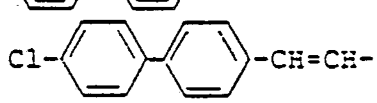 | " |
| 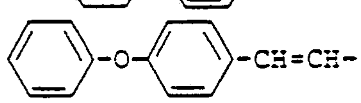 | " |
| 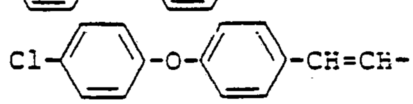 | " |
| 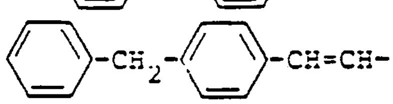 | " |
| 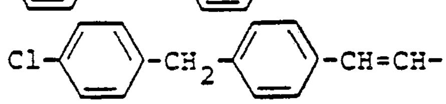 | " |
| 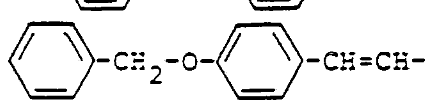 | " |
| 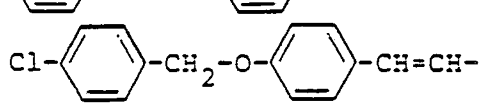 | " |

Tabelle 1 (Fortsetzung)

| $R^1$ | $R^2$ |
|---|---|
| 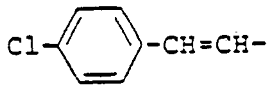 | " |
| 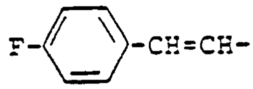 | " |
| 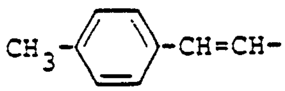 | " |
| 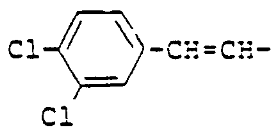 | 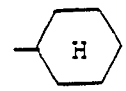 |
| 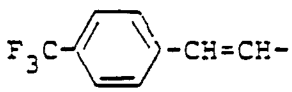 | " |
| 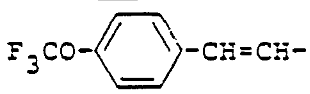 | " |
| 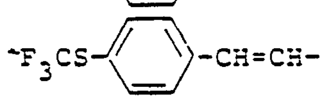 | " |
| 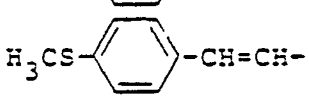 | " |
| 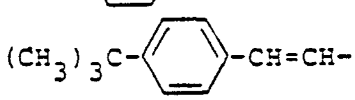 | " |
| 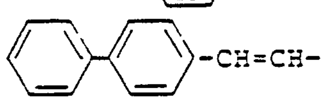 | 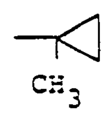 |
| 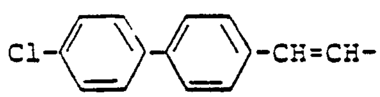 | " |
| 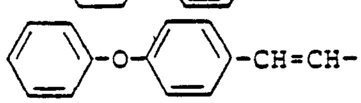 | " |
| 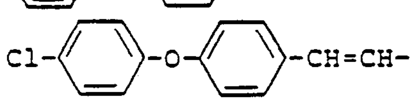 | " |
| 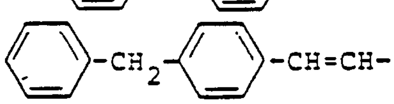 | " |
| 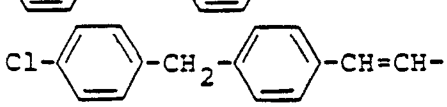 | " |
| 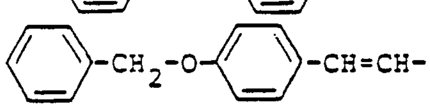 | " |
| 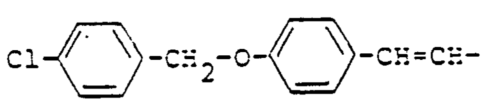 | 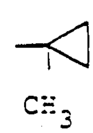 |
| 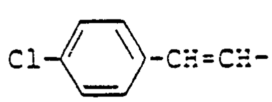 | " |

Tabelle 1 (Fortsetzung)

| $R^1$ | $R^2$ |
|---|---|
| 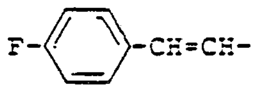 | " |
| 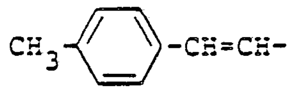 | " |
| 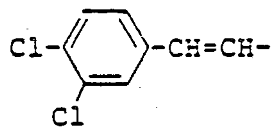 | " |
| 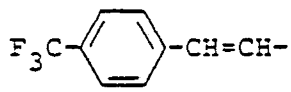 | " |
| 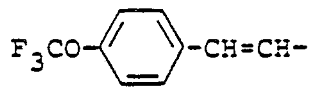 | " |
| 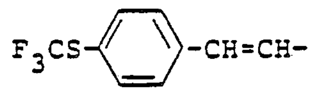 | " |
| 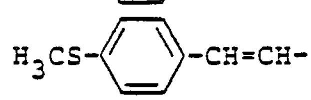 | " |
| 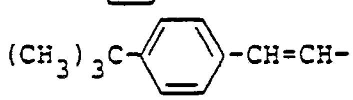 | " |

Die β-Hydroxyethyl-(1,3,4-triazol)-Derivate der Formel (II) lassen sich herstellen, indem man Oxirane der Formel

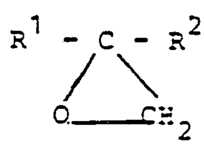

(III)

in welcher

$R^1$ und $R^2$ die oben angegebene Bedeutung haben,

mit 1,3,4-Triazol der Formel

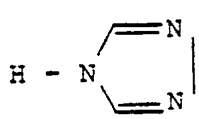

(IV)

in Gegenwart eines Verdünnungsmittels, wie zum Beispiel Methanol, Ethanol, n-Propanol oder Acetonitril, und gegebenenfalls in Gegenwart einer Base, wie zum Beispiel eines Alkalicarbonates oder Alkalialkoholates, bei Temperaturen zwischen 0 °C und 200 °C, vorzugsweise zwischen 60 °C und 150 °C umsetzt. Die Aufarbeitung und die Isolierung der Verbindungen der Formel (II) erfolgen nach üblichen Methoden.

Die bei dem obigen Verfahren zur Herstellung der β-Hydroxyethyl-(1,3,4-triazol)-Derivate als Ausgangsstoffe benötigten Oxirane der Formel (III) sind bekannt oder lassen sich nach bekannten Methoden in einfacher Weise herstellen (vgl. EP-A 40 345, EP-A 46 337 und EP-A 52 424).

Als Basen kommen für die erfindungsgemäße Umsetzung alle üblicherweise verwendbaren anorganischen und organischen Basen in Betracht. Vorzugsweise verwendbar sind Alkalicarbonate, wie Natrium- und Kaliumcarbonat, ferner Alkalihydroxide, Erdalkalihydroxide und -oxide, wie Lithiumhydroxid, Natriumhydroxid, Kaliumhydroxid, Calciumhydroxid und Calciumoxid, weiterhin Alkalialkoholate, wie Natriummethylat, Natriumethylat, Natrium-isobutylat und Kalium-tert.-butylat, und außerdem auch niedere tertiäre Alkylamine, Cycloalkylamine und Aralkylamine, wie insbesondere Triethylamin. Ganz besonders bevorzugt verwendbare Basen sind Natriumhydroxid und Kaliumhydroxid.

Als Verdünnungsmittel sind bei der Durchführung der erfindungsgemäßen Verfahrens aprotische, dipolare Solventien einsetzbar, die gegenüber Basen beständig sind. Bevorzugt sind N,N'-Dimethylformamid, N,N-Dimethylacetamid, Dimethylsulfon, Dimethylsulfoxid, Hexamethylphosphorsäure-triamid, Phospholinoxide, wie 1-Oxo-1-methyl-phospholin, ferner Sulfolan, Tetramethylharnstoff, N-Methylpyrrolidon, N-Methyl-hexahydro-pyridon, 1,3-Dimethylhexahydro-pyrimidon und N-Methyl-ε-caprolactam. Ganz besonders bevorzugt verwendbar ist N-Methyl-pyrrolidon.

Das erfindungsgemäße Verfahren wird vorzugsweise in Gegenwart eines Co-Katalysators durchgeführt. Als Co-Katalysatoren kommen hierbei alle Verbindungen in Frage, die Radikale bilden können. Beispielhaft genannt seien Azo- bis -isobutyronitril und Benzoylperoxid. Es können auch Luft oder Sauerstoff durch den Reaktionsansatz geleitet werden.

In manchen Fällen ist es vorteilhaft, die erfindungsgemäße Umsetzung in Gegenwart einer geringen Menge an Wasser durchzuführen, um so eine bessere Löslichkeit der eingesetzten Base zu erzielen.

Die Reaktionstemperaturen können bei der Durchführung des erfindungsgemäßen Verfahrens in einem größeren Bereich variiert werden. Man arbeitet bei Temperaturen zwischen 50 und 200 °C, vorzugsweise zwischen 70 und 150 °C.

Das erfindungsgemäße Verfahren wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens setzt man auf 1 Mol an β-Hydroxyethyl-(1,3,4-triazol)-Derivat der Formel (II) im allgemeinen 0,01 bis 1 Mol, vorzugsweise 0,1 bis 0,5 Mol an Base und eine ausreichende Menge an aprotischem, dipolaren Verdünnungsmittel ein.

Verwendet man einen Co-Katalysator, so wird dieser in Mengen von 0,01 bis 1 Gewichtsprozent, bezogen auf eingesetztes β-Hydroxyethyl-(1,3,4-triazol)-Derivat der Formel (II), zugegeben. Verwendet man Wasser als Hilfslösungsmittel, so wird dieses nur in Spuren zugesetzt. Im allgemeinen verwendet man Wasser in Mengen von 0,1 bis 2 Gewichtsprozent, bezogen auf die eingesetzte Lösungsmittelmenge. Die Aufarbeitung erfolgt nach üblichen Methoden. Im allgemeinen geht man so vor, daß man das Reaktionsgemisch durch Abdestillieren des Verdünnungsmittels unter vermindertem Druck einengt, den verbleibenden Rückstand mit Wasser und einem mit Wasser wenig mischbaren Lösungsmittel versetzt, die organische Phase abtrennt, trocknet und einengt.

Bei der Durchführung des erfindungsgemäßen Verfahrens können anstelle der Verbindungen der Formel (II) auch Gemische aus Verbindungen der Formeln (I) und (II) eingesetzt werden.

Die nach dem erfindungsgemäßen Verfahren herstellbaren β-Hydroxyethyl-(1,2,4-triazol)-Derivate der Formel (I) sind bekannt (vgl. EP-A 40 345, EP-A 44 605, EP-A 46 337, EP-A 47 594, EP-A 52 424 und DE-A 26 54 890). Sie zeichnen sich durch sehr gute pflanzenwuchsregulierende und fungizide Eigenschaften aus.

Das erfindungsgemäße Verfahren wird durch die nachfolgenden Beispiele veranschaulicht.

Beispiel 1

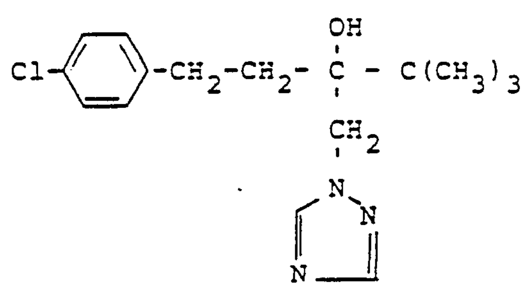

(I - 1)

a) 3 g (9,74 mMol) 1-(4-Chlor-phenyl)-4,4-dimethyl-3-(1,3,4-triazol-1-yl-methyl)-pentan-3-ol wurden in 2 ml N-Methylpyrrolidon suspendiert und mit 40 mg (1 mMol) Natriumhydroxid versetzt. Man erhitzte das Reaktionsgemisch 8 Stunden unter Rühren auf 120 °C, ließ dann abkühlen und arbeitete auf, indem man das Lösungsmittel unter vermindertem Druck abdestillierte, den verbleibenden Rückstand mit Wasser und Methylenchlorid versetzte, dann die organische Phase abtrennte und nach dem Trocknen durch Abziehen des Lösungsmittels einengte. Man erhielt nach Waschen mit wenig Benzin 2,6 g eines Produktes, das gemäß HPLC-Analyse zu 98 % aus 1-(4-Chlor-phenyl)-4,4-dimethyl-3-(1,2,4-triazol-1-yl-methyl)-pentan-3-ol bestand. Die Ausbeute errechnet sich danach zu 84,9 % der Theorie.

Durch HPLC-Analyse wurde nachgewiesen, daß das Produkt nicht durch das entsprechende 1,3,4-Triazol-Derivat verunreinigt ist.

b) In einem weiteren Versuch wurde die oben beschriebene Umsetzung wiederholt, jedoch wurde dem Reaktionsansatz eine Spatelspitze Azo-bis-isobutyronitril zugesetzt.

Man erhielt auf diese Weise 2,95 g eines Produktes, das zu 97 % aus 1-(4-Chlor-phenyl)-4,4-dimethyl-3-(1,2,4-triazol-1-yl-methyl)-pentan-3-ol bestand. Die Ausbeute errechnet sich danach zu 95,4 % der Theorie.

c) 30,8 g (0,1 Mol) 1-(4-Chlor-phenyl)-4,4-dimethyl-3-(1,3,4-triazol-1-yl-methyl)-pentan-3-ol wurden in 125 ml N-Methyl-pyrrolidon suspendiert und mit 4,15 g (0,104 Mol) Natriumhydroxid und 1 ml Wasser versetzt. Man erhitzte das Reaktionsgemisch 4 Stunden unter Rühren auf 120 °C und leitete währenddessen einen trockenen, $CO_2$-freien Luftstrom langsam durch das Reaktionsgemisch. Danach arbeitete man auf, indem man das Lösungsmittel unter vermindertem Druck abdestillierte, den verbleibenden Rückstand mit Wasser und Methylenchlorid versetzte, dann die organische Phase abtrennte und nach dem Trocknen durch Abziehen des Lösungsmittels einengte. Man erhielt auf diese Weise 30,1 g eines Produktes, das zu 96 % aus 1-(4-Chlor-phenyl)-4,4-dimethyl-3-(1,2,4-triazol-1-yl-methyl)-pentan-3-ol bestand. Die Ausbeute errechnet sich danach zu 93,8 % der Theorie.

Durch HPLC-Analyse wurde nachgewiesen, daß das Produkt nicht durch entsprechendes 1,3,4-Triazol-Derivat verunreinigt ist.

Nach der im Beispiel 1 unter (b) angegebenen Methode wurden auch die in der folgenden Tabelle 2 aufgeführten Produkte erhalten.

Tabelle 2

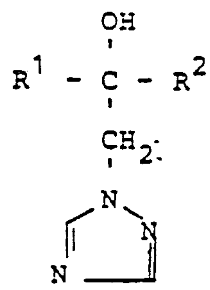

(I)

| Beispiel Nr. | $R^1$ | $R^2$ | Fp. [°C] |
|---|---|---|---|
| 2 | $(CH_3)_3C-$ | H | 83 - 84 |
| 3 | 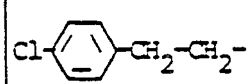 | 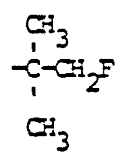 | 110 - 112 |
| 4 | 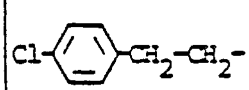 | 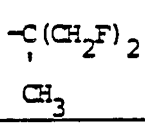 | 122 - 124 |

Herstellung von Ausgangsprodukten

Beispiel (II-1)

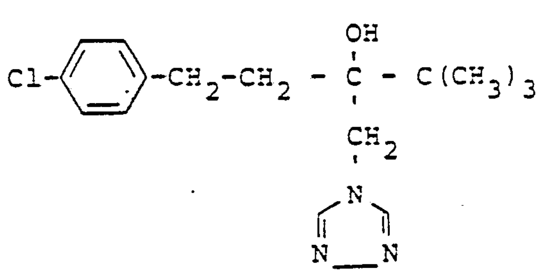

238,5 g (1 Mol) 2-(4-Chlor-phenyl-ethyl)-2-tert.-butyloxiran, 75,9 g (1,1 Mol) 1,3,4-Triazol und 4 g (0,1 Mol) Natriumhydroxid-Plätzchen wurden in 200 ml n-Propanol 30 Stunden unter Rückfluß gerührt. Die

Reaktionslösung wurde unter vermindertem Druck eingedampft, und der Rückstand wurde in der dreifachen Menge Essigester aufgenommen. Es wurde bei 20 °C abfiltriert. Der Rückstand bestand hauptsächlich aus 1-(4-Chlor-phenyl)-4,4-dimethyl-3-(1,3,4-triazol-1-yl-methyl)-pentan-3-ol. Zur weiteren Reinigung wurde aus Aceton umkristallisiert.

Fp. : 188 °C. Ausbeute : 69 g (22,4 %).

Vergleichsbeispiel

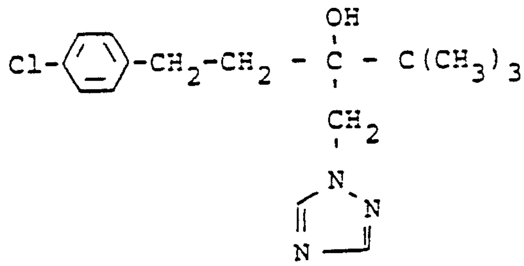

(I-1)

Eine Lösung von 27,1 g (0,1 Mol) eines Produktes, das zu 88 % aus 2-(4-Chlor-phenyl-ethyl)-2-tert.-butyl-oxiran bestand, 8,3 g (0,12 Mol) 1,2,4-Triazol und 0,6 g (0,01 Mol) Kaliumhydroxid in 100 ml n-Propanol wurde 30 Stunden auf 95 °C erhitzt. Danach ließ man abkühlen und engte das Reaktionsgemisch durch Abziehen des Lösungsmittels unter vermindertem Druck ein. Der verbliebene Rückstand wurde in Toluol aufgenommen, die dabei entstehende Suspension wurde filtriert, und das Filtrat wurde durch Abziehen des Lösungsmittels unter vermindertem Druck eingeengt. Der anfallende Rückstand wurde aus Ligroin unkristallisiert. Man erhielt auf diese Weise 30,6 eines Produktes, das gemäß HPLC-Analyse zu 67,4 % aus 1-(4-Chlor-phenyl)-4,4-dimethyl-3-(1,2,4-triazol-1-yl-methyl)-pentan-3-ol bestand. Danach errechnet sich eine Ausbeute von 67 % der Theorie.

**Patentansprüche**

1. Verfahren zur Herstellung von β-Hydroxy-ethyl-(1,2,4-triazol)-Derivaten der Formel

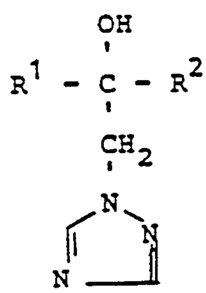

(I)

in welcher

$R^1$ für geradkettiges oder verzweigtes Alkyl mit 1 bis 8 Kohlenstoffatomen, geradkettiges oder verzweigtes Halogenalkyl mit 1 bis 4 Kohlenstoffatomen und 1 bis 5 Halogenatomen, Alkenyl mit 2 bis 8 Kohlenstoffatomen, Alkinyl mit 2 bis 8 Kohlenstoffatomen, gegebenenfalls durch Alkyl mit 1 oder 2 Kohlenstoffatomen substituiertes Cycloalkyl mit 3 bis 7 Kohlenstoffatomen, gegebenenfalls durch Alkyl mit 1 oder 2 Kohlenstoffatomen substituiertes Cycloalkenyl mit 5 bis 8 Kohlenstoffatomen, oder für Phenyl steht, das einfach bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und gegebenenfalls durch Halogen und/oder Alkyl mit 1 bis 4 Kohlenstoffatomen substituiertes Phenylalkoxy mit 1 oder 2 Kohlenstoffatomen im Alkoxyteil

oder

$R^1$ für Phenylalkyl mit 1 bis 4 Kohlenstoffatomen im Alkylteil steht, wobei der Phenylalkyl-Rest im Phenylteil ein- bis dreifach, gleichartig oder verschieden substituiert sein kann durch Halogen,

geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil, oder

$R^1$ für Phenylalkenyl mit 2 bis 4 Kohlenstoffatomen im Alkenylteil steht, wobei der Phenylalkenyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil, oder

$R^1$ für einen gegebenenfalls durch Halogen und/oder Alkyl mit 1 bis 4 Kohlenstoffatomen substituierten heterocyclischen Rest mit 5 bis 7 Ring-Gliedern und 1 bis 3 Heteroatomen steht und

$R^2$ für Wasserstoff, geradkettiges oder verzweigtes Alkyl mit 1 bis 8 Kohlenstoffatomen, geradkettiges oder verzweigtes Halogenalkyl mit 1 bis 4 Kohlenstoffatomen und 1 bis 5 Halogenatomen, Alkenyl mit 2 bis 8 Kohlenstoffatomen, Alkinyl mit 2 bis 8 Kohlenstoffatomen, gegebenenfalls durch Alkyl mit 1 oder 2 Kohlenstoffatomen substituiertes Cycloalkyl mit 3 bis 7 Kohlenstoffatomen, gegebenenfalls durch Alkyl mit 1 oder 2 Kohlenstoffatomen substituiertes Cycloalkenyl mit 5 bis 8 Kohlenstoffatomen steht oder für Phenyl steht, das einfach bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und gegebenenfalls durch Halogen und/oder Alkyl mit 1 bis 4 Kohlenstoffatomen substituiertes Phenylalkoxy mit 1 oder 2 Kohlenstoffatomen im Alkoxyteil oder

$R^2$ für Phenylalkyl mit 1 bis 4 Kohlenstoffatomen im Alkylteil steht, wobei der Phenylalkyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil, oder

$R^2$ für Phenylalkenyl mit 2 bis 4 Kohlenstoffatomen im Alkenylteil steht, wobei der Phenylalkenyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil, oder

$R^2$ für einen gegebenenfalls durch Halogen und/oder Alkyl mit 1 bis 4 Kohlenstoffatomen substituierten heterocyclischen Rest mit 5 bis 7 Ring-Gliedern und 1 bis 3 Heteroatomen steht, dadurch gekennzeichnet, daß man β-Hydroxyethyl-(1,3,4-triazol)-Derivate der Formel

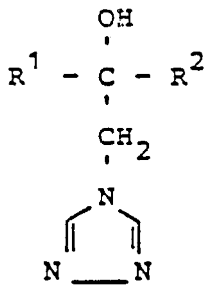

(II)

in welcher
$R^1$ und $R^2$ die oben angegebene Bedeutung haben,
in Gegenwart einer Base und in Gegenwart eines aprotischen, dipolaren Verdünnungsmittels sowie gegebenenfalls in Gegenwart eines Co-Katalysators und gegebenenfalls in Gegenwart von Wasser bei Temperaturen zwischen 50 °C und 200 °C verrührt.

2. Verfahren gemäß Anspruch 1, dadurch gekennzeichnet, daß man als Ausgangssubstanz der Formel (II) das 1-(4-Chlor-phenyl)-4,4-dimethyl-3-(1,3,4-triazol-1-yl-methyl)-pentan-3-ol einsetzt.

3. Verfahren gemäß Anspruch 1, dadurch gekennzeichnet, daß man als Ausgangssubstanz der Formel (II) das 1-(4-Chlor-phenyl)-4-methyl-4-fluormethyl-3-(1,3,4-triazol-1-yl-methyl)-pentan-3-ol einsetzt.

4. Verfahren gemäß Anspruch 1, dadurch gekennzeichnet, daß man als Basen Alkalicarbonate, Alkalihydroxide, Erdalkalihydroxide, Erdalkalioxide, Alkalialkoholate, niedere tertiäre Alkylamine, Cycloalkylamine oder Aralkylamine einsetzt.

5. Verfahren gemäß Anspruch 1, dadurch gekennzeichnet, daß man als aprotische, dipolare Verdünnungsmittel N,N-Dimethylformamid, N,N-Dimethylacetamid, Dimethylsulfon, Dimethylsulfoxid, Hexamethylphosphorsäure-triamid, Phospholinoxid, Sulfolan, Tetramethylharnstoff, N-Methyl-pyrrolidon, N-Methyl-hexahydropyridon, 1,3-Dimethyl-hexahydro-pyrimidon oder N-Methyl-ε-caprolactam einsetzt.

6. Verfahren gemäß Anspruch 1, dadurch gekennzeichnet, daß man als Co-Katalysatoren Verbindungen einsetzt, die Radikale bilden können.

7. Verfahren gemäß Anspruch 1, dadurch gekennzeichnet, daß man als Co-Katalysatoren Azo-bis-isobutyronitril, Benzoylperoxid, Luft oder Sauerstoff einsetzt.

8. Verfahren gemäß Anspruch 1, dadurch gekennzeichnet, daß man Wasser als Hilfslösungsmittel einsetzt.

9. Verfahren gemäß Anspruch 1, dadurch gekennzeichnet, daß man die Umsetzung bei Temperaturen zwischen 70 °C und 150 °C durchführt.

10. Verfahren gemäß Anspruch 1, dadurch gekennzeichnet, daß Ausgangsstoffe (II) eingesetzt werden, die bereits Produkt der Formel (I) enthalten.

## Claims

1. Process for the preparation of β-hydroxy-ethyl-(1,2,4-triazole) derivatives of the formula

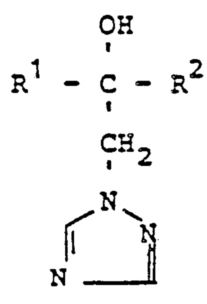

(I)

in which
$R^1$ represents straight-chain or branched alkyl with 1 to 8 carbon atoms, straight-chain or branched halogenoalkyl with 1 to 4 carbon atoms and 1 to 5 halogen atoms, alkenyl with 2 to 8 carbon atoms, alkinyl with 2 to 8 carbon atoms, cycloalkyl which has 3 to 7 carbon atoms and is optionally substituted by alkyl with 1 or 2 carbon atoms, cycloalkenyl which has 5 to 8 carbon atoms and is optionally substituted by alkyl with 1 or 2 carbon atoms, or phenyl which can be mono-, di- or tri-substituted by identical or different

substituents from the group comprising halogen, straight-chain or branched alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms and phenylalkyl which has 1 or 2 carbon atoms in the alkyl part and is optionally substituted by halogen and/or alkyl with 1 to 4 carbon atoms, or represents phenylalkoxy which has 1 or 2 carbon atoms in the alkoxy part and is optionally substituted by halogen and/or alkyl with 1 to 4 carbon atoms, or

$R^1$ represents phenylalkyl with 1 to 4 carbon atoms in the alkyl part, it being possible for the phenylalkyl radical to be mono-, di- or tri-substituted in the phenyl part by identical or different substituents from the group comprising halogen, straight-chain or branched alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, and/or phenylalkoxy which has 1 or 2 carbon atoms in the alkoxy part and is optionally substituted by halogen and/or alkyl with 1 to 4 carbon atoms, or

$R^1$ represents phenylalkenyl with 2 to 4 carbon atoms in the alkenyl part, it being possible for the phenylalkenyl radical to be mono-, di- or trisubstituted in the phenyl part by identical or different substituents from the group comprising halogen, straight-chain or branched alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, and/or phenylalkoxy which has 1 or 2 carbon atoms in the alkoxy part and is optionally substituted by halogen and/or alkyl with 1 to 4 carbon atoms, or

$R^1$ represents a heterocyclic radical which has 5 to 7 ring members and 1 to 3 hetero-atoms and is optionally substituted by halogen and/or alkyl with 1 to 4 carbon atoms and

$R^2$ represents hydrogen, straight-chain or branched alkyl with 1 to 8 carbon atoms, straight-chain or branched halogenoalkyl with 1 to 4 carbon atoms and 1 to 5 halogen atoms, alkenyl with 2 to 8 carbon atoms, alkinyl with 2 to 8 carbon atoms, cycloalkyl which has 3 to 7 carbon atoms and is optionally substituted by alkyl with 1 or 2 carbon atoms, cycloalkenyl which has 5 to 8 carbon atoms and is optionally substituted by alkyl with 1 or 2 carbon atoms, or phenyl which can be mono-, di- or tri-substituted by identical or different substituents from the group comprising halogen, straight-chain or branched alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, phenylalkoxy which has 1 or 2 carbon atoms in the alkoxy part and is optionally substituted by halogen and/or alkyl with 1 to 4 carbon atoms, or

$R^2$ represents phenylalkyl with 1 to 4 carbon atoms in the alkyl part, it being possible for the phenylalkyl radical to be mono-, di- or tri-substituted in the phenyl part by identical or different substituents from the group comprising halogen, straight-chain or branched alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, and/or phenylalkoxy which has 1 or 2 carbon atoms in the alkoxy part and is optionally substituted by halogen and/or alkyl with 1 to 4 carbon atoms, or $R^2$ represents phenylalkenyl with 2 to 4 carbon atoms in the alkenyl part, it being possible for the phenylalkenyl radical to be mono-, di- or trisubstituted in the phenyl part by identical or different substituents from the group comprising halogen, straight-chain or branched alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4

carbon atoms, phenylalkyl which has 1 or 2 carbon atoms in the alkyl part and is optionally substituted by halogen and/or alkyl with 1 to 4 carbon atoms, and/or phenylalkoxy which has 1 or 2 carbon atoms in the alkyl part and is optionally substituted by halogen and/or alkyl with 1 to 4 carbon atoms, or $R^2$ represents a heterocyclic radical which has 5 to 7 ring members and 1 to 3 hetero-atoms and is optionally substituted by halogen and/or alkyl with 1 to 4 carbon atoms,
characterised in that β-hydroxyethyl-(1,3,4-triazole) derivatives of the formula

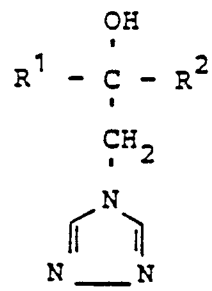

(II)

in which
$R^1$ and $R^2$ have the abovementioned meaning, are stirred in the presence of a base and in the presence of an aprotic, dipolar diluent, and, if appropriate, in the presence of a co-catalyst and, if appropriate, in the presence of water, at temperatures between 50 °C and 200 °C.

2. Process according to Claim 1, characterised in that 1-(4-chloro-phenyl)-4,4-dimethyl-3-(1,3,4-triazol-1-yl-methyl)-pentan-3-ol is used as the starting substance of the formula (II).

3. Process according to Claim 1, characterised in that 1-(4-chloro-phenyl)-4-methyl-4-fluoromethyl-3-(1,3,4-triazol-1-yl-methyl)-pentan-3-ol is used as the starting substance of the formula (II).

4. Process according to Claim 1, characterised in that alkali metal carbonates, alkali metal hydroxides, alkaline earth metal hydroxides, alkaline earth metal oxides, alkali metal alcoholates, lower tertiary alkylamines, cycloalkylamines or aralkylamines are used as the bases.

5. Process according to Claim 1, characterised in that N,N-dimethylformamide, N,N-dimethylacetamide, dimethyl sulphone, dimethylsulphoxide, hexamethylphosphoric acid triamide, phospholine oxide, sulpholane, tetramethylurea, N-methyl-pyrrolidone, N-methyl-hexahydropyridone, 1,3-dimethyl-hexahydro-pyrimidone or N-methyl-ε-caprolactam is used as the aprotic, dipolar diluent.

6. Process according to Claim 1, characterised in that compounds which can form free radicals are used as the co-catalysts.

7. Process according to Claim 1, characterised in that azobisisobutyronitrile, benzoyl peroxide, air or oxygen is used as the co-catalyst.

8. Process according to Claim 1, characterised in that water is used as the auxiliary solvent.

9. Process according to Claim 1, characterised in that the reaction is carried out at temperatures between 70 °C and 150 °C.

10. Process according to Claim 1, characterised in that starting substances (II) which already contain product of the formula (I) are used.

## Revendications

1. Procédé de préparation de dérivés du β-hydroxy-éthyl-(1,2,4-triazole) de formule

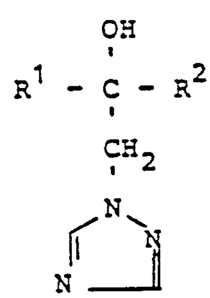

(I)

dans laquelle
$R^1$ représente un groupe alkyle à chaîne droite ou ramifiée en $C_1$-$C_8$, halogénoalkyle à chaîne droite ou ramifiée contenant 1 à 4 atomes de carbone et 1 à 5 atomes d'halogènes, alcényle en $C_2$-$C_8$, alcynyle en $C_2$-$C_8$, cycloalkyle en $C_3$-$C_7$ éventuellement substitué par un groupe alkyle en $C_1$-$C_2$, cycloalcényle en $C_5$-$C_8$ éventuellement substitué par un groupe alkyle en $C_1$-$C_2$, ou un groupe phényle qui peut porter 1 à 3 substituants identiques ou différents choisis parmi les halogènes, les groupes alkyles à chaîne droite ou

ramifiée en $C_1$-$C_4$, cycloalkyles en $C_5$-$C_7$, alcoxy en $C_1$-$C_4$, alkylthio en $C_1$-$C_4$, halogénoalkyles contenant 1 ou 2 atomes de carbone et 1 à 5 atomes d'halogènes, halogénoalcoxy contenant 1 ou 2 atomes de carbone et 1 à 5 atomes d'halogènes, halogénoalkylthio contenant 1 ou 2 atomes de carbone et 1 à 5 atomes d'halogènes, phényles éventuellement substitués par des halogènes et/ou des groupes alkyles en $C_1$-$C_4$, phénoxy éventuellement substitué par des halogènes et/ou des groupes alkyles en $C_1$-$C_4$, phénylalkyles éventuellement substitués par des halogènes et/ou des groupes alkyles en $C_1$-$C_4$ et contenant 1 ou 2 atomes de carbone dans la partie alkyle, et phénylalcoxy éventuellement substitués par des halogènes et/ou des groupes alkyles en $C_1$-$C_4$ et contenant 1 ou 2 atomes de carbone dans la partie alcoxy,

ou bien

$R^1$ représente un groupe phénylalkyle contenant 1 à 4 atomes de carbone dans la partie alkyle, ce groupe phénylalkyle pouvant porter sur la partie phényle 1 à 3 substituants identiques ou différents choisis parmi les halogènes, les groupes alkyles à chaîne droite ou ramifiée en $C_1$-$C_4$, cycloalkyles en $C_5$-$C_7$, alcoxy en $C_1$-$C_4$, alkylthio en $C_1$-$C_4$, halogénoalkyles contenant 1 ou 2 atomes de carbone et 1 à 5 atomes d'halogènes, halogénoalcoxy contenat 1 ou 2 atomes de carbone et 1 à 5 atomes d'halogènes, halogénoalkylthio contenant 1 ou 2 atomes de carbone et 1 à 5 atomes d'halogènes, phényles éventuellement substitués par des halogènes et/ou des groupes alkyles en $C_1$-$C_4$, phénoxy éventuellement substitués par des halogènes et/ou des groupes alkyles en $C_1$-$C_4$, phénylalkyles éventuellement substitués par des halogènes et/ou des groupes alkyles en $C_1$-$C_4$ et contenant 1 ou 2 atomes de carbone dans la partie alkyle, et/ou phénylalcoxy éventuellement substitués par des halogènes et/ou des groupes alkyles en $C_1$-$C_4$ et contenant 1 ou 2 atomes de carbone dans la partie alcoxy

ou bien

$R^1$ représente un groupe phénylalcényle contenant 2 à 4 atomes de carbone dans la partie alcényle, ce groupe phénylalcényle pouvant porter sur la partie phényle 1 à 3 substituants identiques ou différents choisis parmi les halogènes, les groupes alkyles à chaîne droite ou ramifiée en $C_1$-$C_4$, cycloalkyles en $C_5$-$C_7$, alcoxy en $C_1$-$C_4$, alkylthio en $C_1$-$C_4$, halogénoalkyles contenant 1 ou 2 atomes de carbone et 1 à 5 atomes d'halogènes, halogénoalcoxy contenant 1 ou 2 atomes de carbone et 1 à 5 atomes d'halogènes, halogénoalkylthio contenant 1 ou 2 atomes de carbone et 1 à 5 atomes d'halogènes, phényles éventuellement substitués par des halogènes et/ou des groupes alkyles en $C_1$-$C_4$, phénoxy éventuellement substitués par des halogènes et/ou des groupes alkyles en $C_1$-$C_4$, phénylalkyles éventuellement substitués par des halogènes et/ou des groupes alkyles en $C_1$-$C_4$ et contenant 1 ou 2 atomes de carbone dans la partie alkyle et/ou phénylalcoxy éventuellement substitués par des halogènes et/ou des groupes alkyles en $C_1$-$C_4$ et contenant 1 ou 2 atomes de carbone dans la partie alcoxy,

ou bien

$R^1$ représente un radical hétérocyclique contenant de 5 à 7 chaînons cycliques et 1 à 3 hétéroatomes, éventuellement substitué par des halogènes et/ou des groupes alkyles en $C_1$-$C_4$, et

$R^2$ représente l'hydrogène, un groupe alkyle à chaîne droite ou ramifiée en $C_1$-$C_8$, halogénoalkyle à chaîne droite ou ramifiée contenant 1 à 4 atomes de carbone et 1 à 5 atomes d'halogènes, alcényle en $C_2$-$C_8$, alcynyle en $C_2$-$C_8$, cycloalkyle en $C_3$-$C_7$ éventuellement substitué par un groupe alkyle en $C_1$-$C_2$, cycloalcényle en $C_5$-$C_8$ éventuellement substitué par un groupe alkyle en $C_1$-$C_2$ ou un groupe phényle qui peut porter 1 à 3 substituants identiques ou différents choisis parmi les halogènes, les groupes alkyles à chaîne droite ou ramifiée en $C_1$-$C_4$, cycloalkyles en $C_5$-$C_7$, alcoxy en $C_1$-$C_4$, alkylthio en $C_1$-$C_4$, halogénoalkyles contenant 1 ou 2 atomes de carbone et 1 à 5 atomes d'halogènes, halogénoalcoxy contenant 1 ou 2 atomes de carbone et 1 à 5 atomes d'halogènes, halogénoalkylthio contenant 1 ou 2 atomes de carbone et 1 à 5 atomes d'halogènes, phényles éventuellement substitués par des halogènes et/ou des groupes alkyles en $C_1$-$C_4$, phénoxy éventuellement substitués par des halogènes et/ou des groupes alkyles en $C_1$-$C_4$, phénylalkyles éventuellement substitués par des halogènes et/ou des groupes alkyles en $C_1$-$C_4$ et contenant 1 ou 2 atomes de carbone dans la partie alkyle, et phénylalcoxy éventuellement substitués par des halogènes et/ou des groupes alkyles en $C_1$-$C_4$ et contenant 1 ou 2 atomes de carbone dans la partie alcoxy,

ou bien

$R^2$ représente un groupe phénylalkyle contenant 1 à 4 atomes de carbone dans la partie alkyle, ce groupe phénylalkyle pouvant porter sur la partie phényle 1 à 3 substituants identiques ou différents choisis parmi les halogènes, les groupes alkyles à chaîne droite ou ramifiée en $C_1$-$C_4$, cycloalkyles en $C_5$-$C_7$, alcoxy en $C_1$-$C_4$, alkylthio en $C_1$-$C_4$, halogénoalkyles contenant 1 ou 2 atomes de carbone et 1 à 5 atomes d'halogènes, halogénoalcoxy contenant 1 ou 2 atomes de carbone et 1 à 5 atomes d'halogènes, halogénoalkylthio contenant 1 ou 2 atomes de carbone et 1 à 5 atomes d'halogènes, phényles éventuellement substitués par des halogènes et/ou des groupes alkyles en $C_1$-$C_4$, phénoxy éventuellement substitués par des halogènes et/ou des groupes alkyles en $C_1$-$C_4$, phénylalkyles éventuellement substitués par des halogènes et/ou des groupes alkyles en $C_1$-$C_4$ et contenant 1 ou 2 atomes de carbone dans la partie alkyle, et/ou phénylalcoxy éventuellement substitués par des halogènes et/ou des groupes alkyles en $C_1$-$C_4$ et contenant 1 ou 2 atomes de carbone dans la partie alcoxy,

ou bien

$R^2$ représente un groupe phénylalcényle contenant 2 à 4 atomes de carbone dans la partie alcényle, ce groupe phénylalcényle pouvant porter sur la partie phényle 1 à 3 substituants identiques ou différents

choisis parmi les halogènes, les groupes alkyles à chaîne droite ou ramifiée en $C_1$-$C_4$, cycloalkyles en $C_5$-$C_7$, alcoxy en $C_1$-$C_4$, alkylthio en $C_1$-$C_4$, halogénoalkyles contenant 1 ou 2 atomes de carbone et 1 à 5 atomes d'halogènes, halogénoalcoxy contenant 1 ou 2 atomes de carbone et 1 à 5 atomes d'halogènes, halogénoalkylthio contenant 1 ou 2 atomes de carbone et 1 à 5 atomes d'halogènes, phényles éventuellement substitués par des halogènes et/ou des groupes alkyles en $C_1$-$C_4$, phénoxy éventuellement substitués par des halogènes et/ou des groupes alkyles en $C_1$-$C_4$, phénylalkyles éventuellement substitués par des halogènes et/ou des groupes alkyles en $C_1$-$C_4$ et contenant 1 ou 2 atomes de carbone dans la partie alkyle, et/ou phénylalcoxy éventuellement substitué par des halogènes et/ou des groupes alkyles en $C_1$-$C_4$ et contenant 1 ou 2 atomes de carbone dans la partie alcoxy,

ou bien

$R^2$ représente un radical hétérocyclique contenant 5 à 7 chaînons cycliques et 1 à 3 hétéroatomes, éventuellement substitué par des halogènes et/ou des groupes alkyles en $C_1$-$C_4$,

caractérisé en ce que l'on agite des dérivés du β-hydroxyéthyl-(1,3,4-triazole) de formule

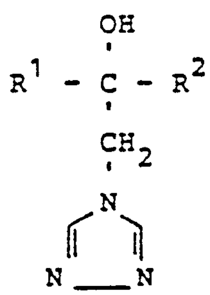

(II)

dans laquelle

$R^1$ et $R^2$ ont les significations indiquées ci-dessus, en présence d'une base et en présence d'un diluant aprotonique dipolaire et le cas échéant en présence d'un co-catalyseur et le cas échéant en présence d'eau, à des températures de 50 à 200 °C.

2. Procédé selon la revendication 1, caractérisé en ce que l'on utilise en tant que produit de départ de formule (II) le 1-(4-chloro-phényl)-4,4-diméthyl-3-(1,3,4-triazole-1-yl-méthyl)-pentane-3-ol.

3. Procédé selon la revendication 1, caractérisé en ce que l'on utilise en tant que produit de départ de formule (II) le 1-(4-chloro-phényl)-4-méthyl-4-fluorométhyl-3-(1,3,4-triazole-1-yl-méthyl)-pentane-3-ol.

4. Procédé selon la revendication 1, caractérisé en ce que l'on utilise en tant que bases de carbonates alcalins, des hydroxydes alcalins, des hydroxydes alcalino-terreux, des oxydes alcalino-terreux, des alcoolates alcalins, des alkylamines tertiaires inférieures, des cycloalkylamines ou des aralkylarmines.

5. Procédé selon la revendication 1, caractérisé en ce que l'on utilise en tant que diluants aprotoniques dipolaires le N,N-diméthylformamide, le N,N-diméthylacétamide, la diméthylsulfone, le diméthylsulfoxyde, l'hexaméthylphosphorotriamide, un oxyde de phospholine, le sulfolan, la tétraméthylurée, la N-méthylpyrrolidone, la N-méthylhexahydropyridone, la 1,3-diméthylhexahydropyrimidone ou le N-méthyl-ε-caprolactame.

6. Procédé selon la revendication 1, caractérisé en ce que l'on utilise en tant que co-catalyseurs des composés capables de former des radicaux libres.

7. Procédé selon la revendication 1, caractérisé en ce que l'on utilise en tant que co-catalyseurs l'azo-bis-isobutyronitrile, le peroxyde de benzoyle, l'air ou l'oxygène.

8. Procédé selon la revendication 1, caractérisé en ce que l'on utilise de l'eau en tant que solvant auxiliaire.

9. Procédé selon la revendication 1, caractérisé en ce que l'on effectue la réaction à des températures de 70 à 150 °C.

10. Procédé selon la revendication 1, caractérisé en ce que l'on utilise des produits de départ de formule (II) qui contiennent déjà un produit de formule (I).